# EUROPEAN PATENT SPECIFICATION

(11) **EP 3 439 647 B1**
(45) Date of publication and mention of the grant of the patent: **22.03.2023**
(21) Application number: 16730030.0
(22) Date of filing: 04.04.2016
(51) Int. Cl.: A61K 31/14, A61K 31/7068, A61K 31/7072, A61K 31/714, A61K 33/18, A61K 33/30, A61P 25/00, A61K 31/202

(54) **COMPOSITION FOR USE IN REDUCING OR PREVENTING GLOBAL DEVELOPMENTAL DELAY IN CHILDREN**
ZUBEREITUNG ZUR VERMINDERUNG ODER VERHINDERUNG VON GLOBALER ENTWICKLUNGSVERZÖGERUNG BEI KINDERN
COMPOSITION POUR LA RÉDUCTION OU LA PRÉVENTION D'UN RETARD DE DÉVELOPPEMENT GLOBAL CHEZ DES ENFANTS

(43) Date of publication of application: 13.02.2019
(73) Proprietor: N.V. Nutricia, 2712 HM Zoetermeer (NL)
(72) Inventor: VAN ELBURG, Ruurd, 3584 CT Utrecht (NL); VOS, Arjen Paul, 3584 CT Utrecht (NL); COUNOTTE, Danielle Stefanie, 3584 CT Utrecht (NL); SMORENBURG, Ana Renée Pascale, 3584 CT Utrecht (NL)
(74) Representative: Nederlandsch Octrooibureau
(86) International application number: PCT/NL2016/050232
(87) International publication number: WO 2017/176109

(56) References cited:
- WO-A1-2012/125020
- WO-A2-01/78530
- Anonymous: "Product Information: Similac NeoSure Similac NeoSure Infant Formula with Iron", , 19 July 2016 (2016-07-19), pages 1-6, XP055313841, Retrieved from the Internet: URL:http://static.abbottnutrition.com/cms- prod/abbottnutrition.com/img/Similac-NeoSu re.pdf [retrieved on 2016-10-25]

## Description

The invention is in the field of medical nutrition and more particularly relates to a composition for use in treating or preventing global developmental delay in children.

### BACKGROUND DESCRIPTION

Brain injury in the perinatal period or in early life of a child has a range of consequences dependent upon the exact timing, location and extent of the brain insult. Brain white matter appears particularly vulnerable to injury in the preterm period, whereas grey matter injury is typically the consequence of acute injury sustained around term birth. It is now recognized, however, that white and grey matter injury occur concurrently in pre-term and term brain injury. Brain injury may result in a global developmental delay. Global developmental delay (GDD) is a term used to indicate that a young child's development is delayed compared to other children, and as such a well-known concept to the skilled person, see DSM-V Diagnostic and statistical manual of mental disorders fifth edition, particularly page 41 therein.

During the last decennium, uridine, choline and omega-3 fatty acids such as DHA have attracted attention as active components in treating cognitive dysfunction, often in context of age-associated neurodegenerative processes. These compounds have been found rate-limiting precursors for membrane phosphatide synthesis. WO 2012/125020 describe such compositions comprising: i) one or more of uridine and cytidine, or salts, phosphates, acylated derivatives or esters thereof; ii) a lipid fraction comprising at least one of docosahexaenoic acid (22:6; DHA), eicosapentaenoic acid (20:5; EPA) and docosapentaenoic acid (22:5; DPA), or esters thereof, in which the lipid fraction comprises less than 2 weight% of linolenic acid (ALA), calculated on the weight of all fatty acids; iii) choline, or salts or esters thereof. In addition, WO 01/78530 discloses infant formulas containing arachidonic acid ("AA", 20:4n-6) and/or docosahexaenoic acid ("DHA", 22:6n-3) as well as their precursor essential fatty acids alpha-linolenic and linoleic acids for providing enhanced neurological development in infants. specifically prematurely born infants

There is a need to further investigate whether dietetic and nutritional intervention helps improving treating or preventing global developmental delay in children.

### SUMMARY OF THE INVENTION

The inventors have observed that upon administering a composition comprising therapeutically effective amounts of (i) one or more of uridine and cytidine, or salts, phosphates, acyl derivatives or esters thereof, (ii) at least one of docosahexaenoic acid (22:6; DHA), eicosapentaenoic acid (20:5; EPA) and docosapentaenoic acid (22:5; DPA), or esters thereof, (iii) choline, or salts or esters thereof, wherein said composition comprises (i) 10 - 200 mg uridine monophosphate (UMP) per 100 kcal or per 100 ml; and (ii)250 - 9000 mg DHA + EPA per 100 kcal or per 100 ml; and (iii) 60 - 350 mg choline per 100 kcal or per 100 ml; and (iv) 1 - 5 µg vitamin B12 or equivalents per 100 kcal or per 100 ml, and (v) per 100 kcal or per 100 ml, 60 - 150 µg of iodine and 5 - 25 mg of zinc, global developmental delay of children up to 5 years of age reduces. The global developmental skills are preferably determined using the Bayley III scales of infant and toddler development as developed in Bayley "Bayley scales of infant and toddler development, third edition" (2006). In a setting of infants or toddlers at risk or suspected of having CP, it was found that the intervention was particularly successful in ameliorating global development delay.

The invention thus pertains to a composition comprising therapeutically effective amounts of (i) one or more of uridine and cytidine, or salts, phosphates, acyl derivatives or esters thereof, (ii) at least one of docosahexaenoic acid (22:6; DHA), eicosapentaenoic acid (20:5; EPA) and docosapentaenoic acid (22:5; DPA), or esters thereof, (iii) choline, or salts or esters thereof, wherein said composition comprises (i) 10 - 200 mg uridine monophosphate (UMP) per 100 kcal or per 100 ml; and (ii) 250 - 9000 mg DHA + EPA per 100 kcal or per 100 ml; and (iii) 60 - 350 mg choline per 100 kcal or per 100 ml; and (iv) 1 - 5 µg vitamin B12 or equivalents per 100 kcal or per 100 ml, and (v) per 100 kcal or per 100 ml , 60 - 150 µg of iodine and 5-25 mg of zinc, for therapeutic use in treating or preventing global developmental delay in children, preferably measured at the scales of the Bayley III test. It is preferred that the composition is administered to said childrenin the form of a supplement to the child's normal diet.

In a preferred embodiment, the child has an early life brain injury, or is at increased risk thereof, preferably a child with suspected cerebral palsy, or a child at increased risk thereof.

### LIST OF FIGURES

Figure 1 shows the cognitive development score assessed with the Bayley III test of children suspected of having CP or diagnosed with CP at baseline, 12 and 24 months of control (placebo) or supplement administration. The result is clinically significant in favor of the supplement group.
Figure 2 shows the language development score assessed with the Bayley III test of children suspected of having CP or diagnosed with CP at baseline, 12 and 24 months of control (placebo) or supplement administration. A clinically significant higher language development score is observed in children receiving the supplement.
Figure 3 shows the motor development score assessed with the Bayley III test of children suspected of having CP or diagnosed with CP at baseline, 12 and 24 months of control (placebo) or supplement administration.

### DETAILED DESCRIPTION OF THE INVENTION

Throughout this application, the following terminology and abbreviations may be used;
Bayley III Test refers to the Bayley Scales of Infant Development version 3 (BSID-III), which is a standard series of measurements used primarily to assess the *motor* (fine and gross), *language* (receptive and expressive), and *cognitive* development of infants and toddlers at ages 16 days till 42 months. In the context of the Bayley III test, age of preterm infants is corrected for gestational age to "term" gestation, to compare their development with other infants. The primary purpose of the Bayley-III is to identify suspected developmental delays in children through the use of norm-referenced scores. Development of older children (> 42 months of age) may be tested with other tests such as the Kaufman Brief Intelligence Test. The Bayley III test consists of a series of developmental play tasks and takes between 45 - 60 minutes to administer. Raw scaled scores ranging from 1 to 19 of successfully completed items are converted to percentile rank scores and to composite scores. These scores are used to determine the child's performance compared with norms taken from typically developing children of their age (in months). In studies including term control groups as a reference population, mean composite BSID-III scores for cognitive development are around 105 for language development around 109 and for motor development around 107 (Bos, A, Developmental Medicine & Child Neurology 2013, 55: 973-979). The composite scores between 85 and 115 are usually accepted as normal development (Bayley N. Bayley Scales of Infant and Toddler Development. 3rd edn. San Antonio, TX: Harcourt Assessment Inc, 2006.)

In the context of the invention, the term "brain injury" refers to a condition in which the brain is damaged by injury caused by an event. As used herein, an "injury" is an alteration in cellular or molecular integrity, activity, level, robustness, state, or other alteration in the brain. The term "perinatal brain injury" refers to brain injuries suffered by a fetus or a neonate. Early-life brain damage refers to brain injuries suffered by an infant in the first trimester up to 1 year of (corrected if born preterm) age. Early life brain injury provides a risk of developing a global developmental delay. Early life brain injury may be caused by one or more of the following: lissencephaly, polymicrogyria, pachygyria, schizencephaly, periventricular leukomalacia, intracranial haemorrhage, intraventricular haemorrhage, basal ganglia lesion(s), thalamus lesion(s), cortico- and subcortical lesion(s), hypoxic-ischemic encephalopathy, (perinatal) stroke, microcephalia vera, hemimegalencephaly, cortical dysplasia, heterotopias, hydranencephaly. In one embodiment, the child suffering from early life brain injury, or at increased risk thereof, is suffering from or at risk of the above pathologies.

A child risking or suffering from global developmental delay ('GDD'), or symptoms thereof, may be child with suspected cerebral palsy (CP), or at increased risk of CP. Children at risk of developing CP can particularly pertain to one of the following groups: (1) born preterm and small for gestational age; (2) born preterm with brain injury; (3) born term but small for gestational age; (4) born term with brain injury, (5) born preterm and small for gestational age with brain injury; and (6) born term but small for gestational age with brain injury.

Cerebral palsy (CP) refers to a group of permanent disorders of the development of movement and posture, causing activity limitation, that are attributed to non-progressive disturbances that occurred in the developing fetal or infant brain. The motor disorders of CP are often accompanied by disturbances of sensation, perception, cognition, communication and behaviour, by epilepsy and by secondary musculoskeletal problems. Consequently, children at risk of cerebral palsy are at risk of global developmental delay.

As used herein, a "child" of the present invention refers to children in early childhood, and, includes, but is not limited to, an individual or subject of any sex that is of an age between the time of delivery to approximately 5 years after delivery. This term includes infants (from 0 to 12 months) and toddlers (between the ages of 12 and 42 months of age). In one embodiment, the preferred targeted children are preferably up to 5 years, preferably up to 4 years of age, preferably up to 42 months of age.

Global developmental delay (GDD) is a DSM-V diagnosis for children under the age of 5 years, wherein a child fails to meet expected developmental milestones, in several areas of intellectual functioning, that are associated with normal development. The diagnosis is used for individuals who are unable to undergo systematic assessments of intellectual functioning, including children who are too young to participate in standardized testing. Cognitive impairment is therefore often associated with a history of delayed developmental milestones. The Bayley Scales of Infant Development version III (BSID-III) is a tool to measure development in young children and can help to identify children with global developmental delay in children between 16 days and 42 months. Global developmental delay is defined as a score lower than 85 on the Bayley III scale.

Preterm infant means a subject born before 37 weeks gestational age. Within the group of preterms, preterm infants can be further differentiated in children born very preterm (28 to 32 weeks of gestational age) and extreme preterms (born before 28 weeks of gestational age). The term "preterm infant" is used interchangeably with "premature infant". Premature infants are at greater risk for cerebral palsy, delays in development, hearing problems, and problems seeing. These risks are greater the earlier a baby is born.

"Small for gestational age" means an infant born smaller in size than normal for the gestational age, most commonly defined as a weight below the 10th percentile for the gestational age.

Term infant refers to an infant born between 37 and 42 weeks gestational age.

In one aspect of the present invention, (i) - (v)are part of a composition according to the invention which is used as a pharmaceutical product comprising one or more pharmaceutically acceptable carrier materials.

In a preferred aspect of the present invention, (i) - (v)are part of a composition which is used as a nutritional product, for example as a nutritional supplement, e.g., as an additive to a normal diet, as a fortifier, to add to a normal diet, or as a complete nutrition. Advantageously such supplement could be added in addition to the normal nutritional needs for that particular age, and the dosage could be adjusted thereto. A representation of the preferred amounts of the components (i) - (v)in terms of the child's body weight is in accordance therewith.

A first aspect of the invention provides for a composition comprising therapeutically effective amounts of (i) one or more of uridine and cytidine, or salts, phosphates, acyl derivatives or esters thereof, (ii) at least one of docosahexaenoic acid (22:6; DHA), eicosapentaenoic acid (20:5; EPA) and docosapentaenoic acid (22:5; DPA), or esters thereof, (iii) choline, or salts or esters thereof, wherein said composition comprises (i) 10 - 200 mg uridine monophosphate (UMP) per 100 kcal or per 100 ml; and (ii) 250 - 9000 mg DHA + EPA per 100 kcal or per 100 ml; and (iii) 60 - 350 mg choline per 100 kcal or per 100 ml; and (iv) 1 - 5 µg vitamin B12 or equivalents per 100 kcal or per 100 ml, and (v) per 100 kcal or per 100 ml, 60 - 150 µg of iodine and 5-25 mg of zinc for therapeutically treating or preventing global developmental delay in a child, by administering said composition to said child.

In one embodiment, neurodevelopment, such as at an (corrected) age of 12 months or 24 months, is assessed based on a score from Bayley Scales of Infant and Toddler Development (Bayley III). For the Bayley III test, the age of preterm infants is corrected for term gestational age to allow for a true comparison of development. Thus, at e.g. 12 months (corrected) age, neurodevelopment of an infant may be assessed and any neurodevelopmental delay may be detected. In one embodiment, said Bayley III composite score is based on assessment(s) of one or more of cognitive, motor, and language function(s) of the infant. It should be understood that, in the context of the present invention, these infants or children are targeted which are at increased risk of or suffering from global developmental delay

In one embodiment, the targeted child having a global developmental delay has a Bayley III cognitive composite score of less than 85. In some instances, the global developmental delay is considered severe and in such cases said Bayley III language score may be less than 70.

In one embodiment, the invention involves use of the composition according to the invention in a child at risk of early life brain injury, particularly children with suspected cerebral palsy, or at increased risk thereof, preferably a child identified having a Bayley III composite score of at least 85.

According to another embodiment, the composition according to the invention is administered, preferably as a supplement or fortifier, to a child at risk of early life brain injury, particularly children with suspected cerebral palsy, or at increased risk thereof, at least daily for a period of at least 3 months, preferably at least 6 months, at least 9 months, at least 12 months, at least 15 months, at least 18 months, at least 21 months and more preferably at least 24 months. In one embodiment, the composition is a daily dose unit, suitable for administration once a day.

In one embodiment the child is 0 to 12 years of age, preferably 0 to 5 years, preferably 0 to 4 years, more preferably 0 to 42 months.

Throughout the specification and claims, the dosages of the active components are expressed in terms of body weight. These can for instance be calculated based on WHO-provided infant/children average growth charts. However, it is preferred to administer the composition according to the invention with the amounts as expressed throughout the specification and claims capped at a maximum of 30 kg body weight. As an example, a child weighing 36 kilos (the average weight of an 11-year old child) of may receive the preferred dosage for a 30 kg body weight child.

Preferably, the composition according to the invention comprises UMP and CMP.

Preferably, the composition according to the invention further comprises arachidonic acid.

In an embodiment of the invention a daily dose of the composition, according to the invention preferably comprises between 10 to 50 µg per kg body weight iodine, preferably 12 to 48 µg per kg body weight, preferably 14 to 48 µg per kg body weight, more preferably 14 to 20 µg per kg body weight, more preferably 15 to 20 µg per kg body weight of iodine. In an embodiment the total daily dose of iodine does not exceed 450 µg. Preferably the daily dose of the composition and method according to the invention preferably comprises between 0.5 to 2.5 mg per kg body weight zinc, preferably between 0.6 and 2.2 mg per kg body weight more preferably 0.6 to 1.0 mg per kg body weight of zinc, and even more preferably 0.7 to 1.0 mg per kg body weight. In an embodiment the total daily dose of zinc does not exceed 18 mg.

Preferably the daily dose of vitamin B12 in the composition according to the invention preferably is in the range of 0.05 to 1.0 µg per kg body weight, preferably 0.075 to 0.75 µg per kg body weight, preferably 0.09 to 0.5 µg per kg body weight, more preferably 0.09 - 0.2 µg per kg body weight, even more preferably 0.1 - 0.2 µg per kg body weight of vitamin B12.

Preferably, the compositionis provided as a complete nutritional product (comprising carbohydrates, fats and protein) or as a nutritional supplement. Preferably the composition is a complete nutritional source by providing balanced amounts of all recommended nutrients .

The composition of the invention is an enteral composition, intended for oral administration. It is preferably administered in liquid form. In a preferred embodiment, the composition is a powder to be reconstituted with water prior to use. In an embodiment the enteral composition of the invention is an infant formula. In a preferred embodiment, the enteral composition of the invention is an infant formula, for use as sole source of nutrition. In a preferred embodiment, the composition is a nutritional supplement for use to supplement other dietary intake.

In another preferred embodiment, the enteral composition is a nutritional supplement or fortifier. In yet another embodiment the enteral composition of the invention is a tube feed.

In an embodiment the composition is a powder or a liquid composition containing between 50 and 200 kcal per 100 ml, preferably between 65 and 150 kcal per 100 ml, more preferably between 75 and 100 kcal per 100 ml.

Further details of a composition of the invention are provided here below.

### ω-3 LC-PUFAs

The composition of the invention comprises therapeutically effective amounts of at least one omega-3 long-chain polyunsaturated fatty acid (LC PUFA; having a chain length of 18 and more carbon atoms) selected from the group consisting of docosahexaenoic acid (22:6; DHA), and eicosapentaenoic acid (20:5; EPA), wherein said composition comprises 250 - 9000 mg DHA + EPA per 100 kcal or per 100 ml. The composition of the invention can further comprise therapeutically effective amounts of docosapentaenoic acid (22:5 w3, DPA).

The LCPUFAs (DHA, EPA and/or DPA) are preferably provided as triglycerides, diglycerides, monoglycerides, free fatty acids or their salts or esters, phospholipids, lysophospholipids, glycerol ethers, lipoproteins, ceramides, glycolipids or combinations thereof. Preferably, the present composition comprises at least DHA in triglyceride form. Suitable ω-3 LCPUFA and/or DHA sources include tuna oil, (other) fish oils, DHA-rich alkyl esters, algae oil, egg yolk, or phospholipids enriched with ω-3 LCPUFA e.g. phosphatidylserine-DHA. Preferably, a composition according to the invention comprises fish oil providing the omega-3 LCPUFA(s). Another particularly suitable source for the omega-3 LCPUFA(s) is algae oil.

Preferably, the total daily dosage of DHA+EPA+DPA taken together is in the range of 35 - 75 mg/kg body weight, more preferably 40 - 72.5 mg/kg body weight, even more preferably 43.5 - 72.5 mg/kg body weight, most preferably 43.5 - 60 mg/kg body weight, particularly 43 - 49.5 mg/kg body weight, whether or not the three (DHA, EPA and/or DPA) are present in the composition according to the invention altogether. Preferably, these amounts are based on the total sum of DHA and EPA if present. In a preferred embodiment the total daily dose of DHA+EPA+DPA provided by the composition is at most 1500 mg.

Preferably, the composition comprises at least DHA. DHA is preferably administered in a daily amount of at least 32.5 - 65 mg/kg body weight, more preferably 35 - 62.0 mg/kg body weight, even more preferably 36-60 mg/kg body weight, most preferably 36-50 mg/kg body weight, particularly 36-41 mg/kg body weight. In a preferred embodiment the total daily dose of DHA provided by the composition is at most 1200 mg.

In terms of the composition, the proportion of ω-3 LCPUFA (more preferably DHA+EPA+DPA, most preferably DHA+EPA) of the total fat in the composition is preferably 1 to 95 wt%, more preferably 1 to 50 wt%, more preferably 1 to 30 wt% of the total fat. The present composition preferably comprises 0.5 to 95 wt% DHA based on total fat, preferably 0.5 to 75 wt% DHA based on total fatty acids, more preferably 10 to 60 wt%, even more preferably 10 - 50 wt%, more preferably 1-40 wt% based on total fat of the composition. The present composition preferably comprises 0.1 to 95 wt% EPA based on total fat, preferably 0.5 to 75 wt% EPA, even more preferably 0.5 - 50 wt%, more preferably 0.5 - 25 wt%, most preferably 0.5 - 15 wt%, based on total fat of the composition.

In the composition of the invention, the ratio of the weight of DHA to EPA is preferably larger than 1, more preferably 2:1 to 10:1, more preferably 3:1 to 7.5:1. The ratios take into account and optimize the balance between DHA and precursors thereof to ensure optimal effectiveness while maintaining low-volume formulations.

The DHA/AA weight ratio in the present composition is preferably of at least 2, more preferably at least 5, preferably at least 6. If AA is administered, it preferably amounts to a daily amount of 2 - 35 mg/kg body weight, preferably 3 - 33 mg/kg body weight, more preferably 4 - 32.5 mg/kg body weight, even more preferably 4 - 8 mg/kg body weight.

### Uridine, cytidine and/or equivalents thereof

The composition according to the invention comprises therapeutically effective amounts of one or more of uridine, cytidine and/or an equivalent thereof, including salts, phosphates, acyl derivatives and/or esters. The composition comprises at least 10 - 200 mg uridine monophosphate (UMP) per 100 kcal or per 100 ml.

In one embodiment, cytidine, CMP, citicoline (CDP-choline) may also be applied in addition to UMP.

The inclusion of UMP in the present composition enables a high effectivity or efficacy at the lowest dosage and/or the administration of a low volume to the subject. Preferably at least 50 weight% of the uridine in the present composition is provided by UMP, more preferably at least 75 weight%, most preferably at least 95 weight%. Doses administered are given as UMP. The amount of uracil sources can be calculated taking the molar equivalent to the UMP amount (molecular weight 324 Dalton).

In one embodiment, the present composition of the invention comprises the sum of uridine and cytidine, including equivalents, in amounts of daily dosage of 3.0 - 8.5 mg/kg body weight, preferably 3.4 - 8.0 mg/kg body weight, more preferably 3.6 - 7.5 mg/kg body weight, even more preferably 3.6 - 5 mg/kg body weight, most preferably 3.6 - 4.4 mg/kg body weight.

The present composition preferably comprises uridine (the cumulative amount of uridine, deoxyuridine, uridine phosphates, nucleobase uracil and acylated uridine derivatives). The composition is administered with a preferred daily dosage of uridine would amount to 1.0 - 4.0 mg per kg body weight, preferably 1.0 - 3.5 mg per kg body weight, more preferably 1.5 - 3.0 mg per kg body weight, more preferably 1.8 - 3.0 mg/kg body weight, even more preferably 1.8 - 2.0 mg/kg body weight. In a preferred embodiment the total daily dose of uridine (equivalents) provided by the composition is at most 625 mg.

In an embodiment, the present composition according to the invention comprise (therapeutically) effective amounts of cytidine and/or an equivalent thereof, including salts, phosphates, acyl derivatives and/or esters. Preferably, a cytidine phosphate is selected from the group consisting of cytidine monophosphate (CMP), cytidine diphosphate (CDP) and cytidine triphosphate (CTP). The preferred cumulative daily dosage of cytidine amounts to 1.0 - 6.0 mg per kg body weight, preferably 1.0 - 5.0 mg per kg body weight, more preferably 1.5 - 4.8 mg per kg body weight, more preferably 1.8 - 4.5 mg/kg body weight, even more preferably 1.8 - 2.2 mg/kg body weight. In a preferred embodiment, these amounts are in addition to the amounts of uridine here above.

### Choline

The composition according to the present invention comprises therapeutically effective amounts of choline, a choline salt and/or choline ester, wherein said composition comprises 60 - 350 mg choline per 100 kcal or per 100 ml. Herein, the term 'choline' shall be considered to encompass all these equivalents. Choline salts are preferred. The choline salt is preferably selected from choline chloride, choline bitartrate, or choline stearate. The choline ester is preferably selected from the group consisting of phosphatidylcholine and lyso-phosphatidylcholine. The present composition is preferably administered in a daily dosage of preferably 5 - 40 mg per kg body weight per day. The daily amount of choline would amount preferably to 7.5 to 35 mg, more preferably 8 to 31 mg, more preferably 9-31 mg per kg body weight, most preferably 9 to 14 mg choline per kg body weight. The above numbers are based on choline, the amounts of choline equivalents or sources can be calculated taking the molar equivalent to choline into account, based on the molar mass of 104 g/mol choline. In a preferred embodiment the total daily dose of choline (equivalents) provided by the composition is at most 400 mg.

### B Vitamins

The present composition according to the invention comprises therapeutically effective amounts of vitamin B12 (cobalamins), wherein said composition comprises 1 - 5 µg vitamin B12 or equivalents per 100 kcal or per 100 ml. Functional equivalents are encompassed within these terms.

Vitamin B 12 is preferably present in an amount to provide a daily dosage in the range of 0.05 to 1.0 µg per kg body weight, preferably 0.075 to 0.75 µg per kg body weight , preferably 0.09 to 0.5 µg per kg body weight, more preferably 0.09 - 0.2 µg per kg body weight. The term "vitamin B12" incorporates all cobalbumin equivalents known in the art. In a preferred embodiment the total daily dose of vitamin B12 (equivalents) provided by the composition is at most 5 µg.

In the composition of the invention, zinc is provided in an amount of 5 - 25 mg of zinc per 100 kcal or per 100 ml..

In the composition of the invention, iodine is provided in an amount of 60 - 150 µg of iodine per 100 kcal or per 100 ml..

The composition may further comprise a digestible carbohydrate fraction, preferably including a source of lactose and a source of polysaccharide.

In one aspect of the invention, the compositions either as complete nutrition or as a supplement as detailed above are intended for use in the treatment or prevention of GDD.

### EXAMPLES

For a more complete understanding of the present disclosure, reference is now made to the following examples taken in conjunction with the accompanying drawings.

### Example 1 - clinical trial study with children suspected of or diagnosed with cerebral palsy

In a two-year blind randomized placebo-controlled trial it was investigated whether postnatal dietetic and nutritional intervention, with neurotrophic supplementation according to the invention (table 1A and B), improves growth and neurodevelopmental outcomes in children who are between 1 and 18 months of age at inclusion in the study and have a suspected or confirmed clinical diagnosis of Cerebral Palsy according to the following definition. Children included in the study with suspected or confirmed CP suffer from a group of permanent disorders of the development of movement and posture, causing activity limitation, that are attributed to non-progressive disturbances that occurred in the developing fetal or infant brain. The motor disorders of cerebral palsy are often accompanied by disturbances of sensation, perception, cognition, communication, and behavior, by epilepsy, and by secondary musculoskeletal problems. Children with progressive neurological degenerative conditions, such low hearing they cannot complete assessment with the Bayley scales, children with gastrointestinal disease which significantly impairs absorption as well as children from parents that were considered unable to follow the study protocol were excluded from participation in the study.

Neurological development was assessed using the Bayley Scale of Infant Development (BSID III), at baseline, 12 and 24 months after enrollment in the study. This score is corrected for the age of the child in months at the time of the scoring.

Both groups received:
- Fortnightly dietetic review (in person or by telephone)
- Advice regarding feeding and optimizing both dietary macro (glucose polymers and/or long chain triglycerides) and micronutrient intake.
- Both groups received a measured feed supplement (neurotrophic factors or placebo as described in Table 1A and 1B) to add to feed/food once a day.

**Table 1A. Nutritional values of the main ingredients of the neurotrophic feed supplement and control or placebo supplement and 1B feeding schedule for children enrolled in the clinical trial receiving the study products.**

**Table 1A.**

| *Nutrients* | **nutritional value active supplement** (501 kcal /100 g) | **nutritional value control supplement** (503 kcal/100 g) |
|---|---|---|
| **MACRO NUTRIENTS** | | |
| Energy | 501kcal/ 100 g | 503 kcal/100g |
| Protein | 2.2 g / 100 kcal | 2.2 g/100 kcal |
| Whey | 1.3 g / 100 kcal | 1.3 g/100 kcal |
| **Fat** | 5.0 g / 100 kcal | 5.0 g/100 kcal |
| Linoleic acid (LA) | 534 mg / 100 kcal | 693 mg/100 kcal |
| Alpha-linolenic acid | 77 mg / 100 kcal | 122 mg/100 kcal |
| Mysteric acid | 63 mg / 100 kcal | 29 mg / 100 kcal |
| Palmitoleic acid | 74 mg / 100 kcal | 8.2 mg / 100 kcal |
| Oleic acid | 1917 mg / 100 kcal | 2637 mg / 100 kcal |
| ALA | 77 mg / 100 kcal | 122 mg / 100 kcal |
| AA | 42 mg /100 kcal | 20 mg/100 kcal |
| EPA | 77 mg / 100 kcal | 0.8 mg/100 kcal |
| DHA | 377 mg /100 kcal | 3.7 mg/100 kcal |
| (n-6)/(n-3) ratio | 5.4 | 1.1 |
| LA/ALA ratio | 5.7 | 6.9 |
| **Carbohydrates** | 11.6 g /100 kcal | 11.7 g/100 kcal |
| Sugars | 8.9 g / 100 kcal | 9.1 g/100 kcal |
| Milk fat | 0.1 g / 100 kcal | 0.1 g / 100 kcal |

| **MINERALS** | | |
|---|---|---|
| Na | 25 mg / 100 kcal | 25 mg / 100 kcal |
| K | 93 mg / 100 kcal | 116mg / 100 kcal |
| Cl | 63 mg / 100 kcal | 63 mg / 100 kcal |
| Ca | 63 mg / 100 kcal | 63 mg / 100 kcal |
| P | 58 mg / 100 kcal | 40 mg / 100 kcal |
| Mg | 6.3 mg / 100 kcal | 6.3 mg / 100 kcal |

| **TRACE ELEMENTS** | | |
|---|---|---|
| Fe | 0.62 mg / 100 kcal | 0.63 mg / 100 kcal |
| Zn | 7.5 mg / 100 kcal | 0.63 mg / 100 kcal |
| Cu | 25 µg / 100 kcal | 25 µg / 100 kcal |
| Mn | 4.4 µg / 100 kcal | 4.4 µg / 100 kcal |
| Se | 1.3 µg / 100 kcal | 1.3 µg / 100 kcal |
| I | 150 µg / 100 kcal | 6.2 µg / 100 kcal |

| **VITAMINS** | | |
|---|---|---|
| Vitamin A | 75 µg RE/ 100 kcal | 75 µg RE/ 100 kcal |
| Vitamin D3 | 1.8 µg / 100 kcal | 1.8 µg / 100 kcal |
| Vitamin E | 1.4 mg α-TE / 100 kcal | 1.6 mg α-TE / 100 kcal |
| Vitamin K1 | 6.0 µg / 100 kcal | 6.7 µg / 100 kcal |
| Thiamin (B1) | 50 µg / 100 kcal | 50 µg / 100 kcal |
| Riboflavin (B2) | 110 µg / 100 kcal | 110 µg / 100 kcal |
| Niacin (B3) | 1.0 mg NE/ 100 kcal | 1.5 mg NE/ 100 kcal |
| Pantothenic acid (B5) | 375 µg / 100 kcal | 375 µg / 100 kcal |
| Vitamin B6 | 44 µg / 100 kcal | 44 µg / 100 kcal |
| Folic acid | 5.0 µg / 100 kcal | 5.0 µg / 100 kcal |
| Vitamin B12 | 1.2 µg / 100 kcal | 0.17 µg / 100 kcal |
| Biotin | 2.0 µg / 100 kcal | 2.0 µg / 100 kcal |
| Vitamin C | 10 mg / 100 kcal | 10 mg / 100 kcal |
| Choline | 105 mg / 100 kcal | 14 mg / 100 kcal |
| Taurine | 0.41 mg / 100 kcal | 0.41 mg / 100 kcal |
| L-Carnitine | 1.8 mg / 100 kcal | 1.8 mg / 100 kcal |
| Inositol | 7.2 mg / 100 kcal | 6.2 mg / 100 kcal |
| UMP | 18 mg / 100 kcal | 0 mg / 100 kcal |
| CMP | 18 mg / 100 kcal | 0 mg / 100 kcal |
| Total nucleotides | 36 mg / 100 kcal | 0 mg / 100 kcal |

**Table 1B.**

| **Body weight (kg)** | Required study product (gram / day) |
|---|---|
| < 1 | 2 |
| 1-1.25 | 2.5 |
| 1.25-1.5 | 3 |
| 1.5-1.75 | 3.5 |
| 1.75-2 | 4 |
| 2-2.25 | 4.5 |
| 2.25-2.5 | 5 |
| 2.5-3 | 6 |
| 3-3.5 | 8 |
| 3.5-4 | 9 |
| 4-5 | 11 |
| 5-6 | 13 |
| 6-7 | 14 |
| 7-8 | 16 |
| 8-9 | 18 |
| 9-10 | 20 |
| 10-11 | 22 |
| 11-12 | 24 |
| > 12 | 24 |

The treatment groups were balanced for the minimization factors: gender, cerebral palsy type, visual impairment and corrected age at time of recruitment. There were fewer subjects in the youngest (1-4 months) category for corrected age at time of recruitment than in the middle (6- 12 months) category in the placebo group whereas there were equal numbers of subjects who were 1-4 months and 6- 12 months at time of recruitment in the supplement group. The median gestational age at birth was 3 weeks lower in the supplement group.

Table 2 summarizes the characteristics of the groups assigned to either placebo or supplement at randomization.

**Table 2. Characteristics of children at baseline randomized to placebo or supplement.**

| | Placebo | Supplement |
|---|---|---|
| | N or mean (SD) or median (LQ, UQ) | N or mean (SD) or median (LQ, UQ) |
| Numbers assigned treatment | 20 | 20 |
| Cerebral Palsy type: 4 limb involvement / other | 8/12 | 7/13 |
| Corrected age at time of recruitment: 1- 5 months / 6-12 months / 13-18 months | 2/8/10 | 5/5/10 |
| Sex: female / male | 6/14 | 6/14 |
| Gestational age at birth | 38.0 (32.5, 41.0) | 35.0 (28.0, 38.5) |
| Maternal educational qualification: GCSE / A level / College+ | 7/8/5 | 9/4/5 * |
| Bayley cognitive score | 75.3 (17.5) | 72.5 (15.5) |
| Bayley language score | 74.7 (15.7) | 77.7 (13.9) |
| Bayley motor score | 63.4 (19.4) | 65.7 (16.9) |

| | | |
|---|---|---|
| * Information missing for 2 subjects that received supplement. | | |

The primary analysis was based on the intention-to-treat and involved all subjects who were randomly assigned and had information on Bayley cognitive score at baseline and at least one other time point (12 months or 24 months). Thus data from 32 subjects were available for the intention-to-treat analysis. Two subjects were considered protocol violators. One subject withdrew before supplementation but also has data at 12 months so was excluded from the per- protocol analysis. One subject was randomized to receive placebo but actually received supplement for the 2 years of follow-up so was analysed as having received supplement in the per-protocol analysis. Consequently 31 subjects remained in the per- protocol analysis that will be discussed below.

At baseline, 12 and 24 months all children participating in the follow-up underwent Bayley III assessment to assess their language, cognitive and motor skills.

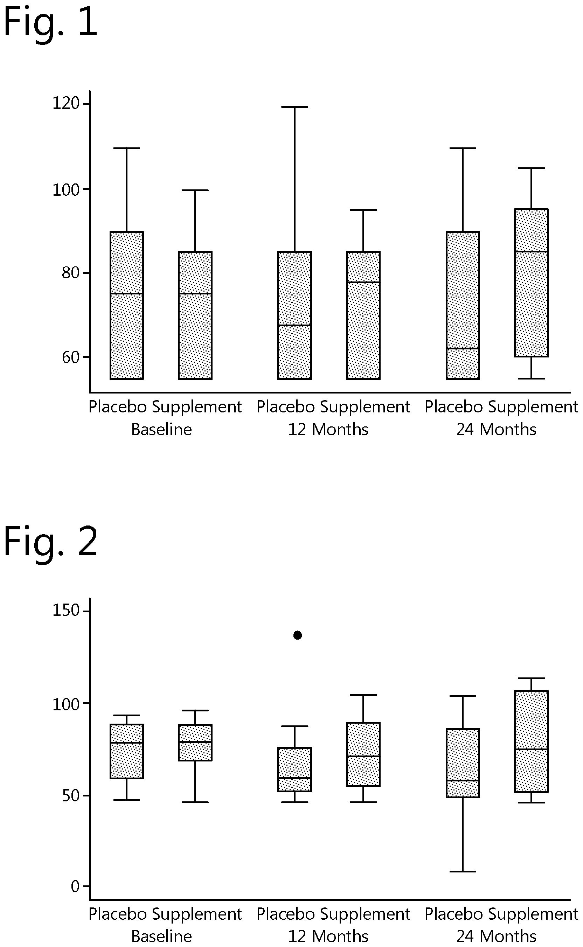

The median Cognitive composite Bayley III scores for the children suspected of or diagnosed with cerebral palsy enrolled in the study are shown in figure 1; table 3 shows the mean cognitive composite scores for these children. The scores are determined at baseline, 12 months and 24 months after enrollment in the study for both the group receiving placebo and the group receiving the supplement. An increase in Bayley III cognitive score was observed for children receiving the supplement as compared to children receiving the placebo. Table 4 describes the distribution of children scoring < 85 or > 85 on the Bayley III cognitive composite score. In the group of children that received the supplement according to the present invention both at 12 and at 24 months an increased number of children with a cognitive development score ≥ 85 is observed.

**Table 3 Bayley III cognitive composite score**

| *Visit* | *Numbers included*^{∗} | | *Mean (SD)*^{∗} | | *Difference in means (95% CI)*^{∗}# |
|---|---|---|---|---|---|
| | *Placebo* | *Supplement* | *Placebo* | *Supplement* | |
| Baseline | 18 | 14 | 73.6 (17.6) | 71.4 (14.7) | |
| 12 month | 18 | 14 | 71.1 (18.8) | 75.0 (15.8) | 4.6 (-2.4, 11.6) |
| 24 month | 16 | 13 | 72.2 (19.8) | 77.7 (19.2) | 4.5 (-2.7, 11.8) |

| | | | | | |
|---|---|---|---|---|---|
| * All subjects included in analysis have a measurement for Bayley cognitive score at baseline and at least one other time point; # adjusted for baseline measurement, treatment, visit, interaction between treatment and visit, and the minimisation factors gender, cerebral palsy type, visual impairment and corrected age at time of recruitment. | | | | | |

**Table 4 Distribution of Bayley III cognitive composite score**

| | *Placebo* | *Supplement* |
|---|---|---|
| | *Numbers <85* / *numbers* ≥ *85* | *Numbers* <*85* / *numbers* ≥ *85* |
| Baseline | 12 / 8 | 15 / 5 |
| 12 month | 13 / 7 | 8 / 12 |
| 24 month | 10 / 10 | 6 / 14 |

The median performance of the same children on language development is shown in figure 2, mean language composite scores are shown in table 5. An improved language development score was observed over time in children that received the nutritional supplement as compared to the placebo group

**Table 5. Bayley III Language composite score**

| *Visit* | *Numbers included*^{∗} | | *Mean (SD)*^{∗} | | *Difference in means (95% CI)*^{∗}# |
|---|---|---|---|---|---|
| | *Placebo* | *Supplement* | *Placebo* | *Supplement* | |
| Baseline | 18 | 14 | 73.2 (15.9) | 77.1 (12.9) | |
| 12 month | 18 | 14 | 68.2 (21.0) | 74.8 (19.8) | 5.0 (-5.1, 15.1) |
| 24 month | 16 | 13 | 66.1 (24.9) | 78.5 (25.6) | 10.1 (-0.4,20.6) |

| | | | | | |
|---|---|---|---|---|---|
| * All subjects included in analysis have a measurement for Bayley language score at baseline and at least one other time point; # adjusted for baseline measurement, treatment, visit, interaction between treatment and visit, and the minimisation factors gender, cerebral palsy type, visual impairment and corrected age at time of recruitment. | | | | | |

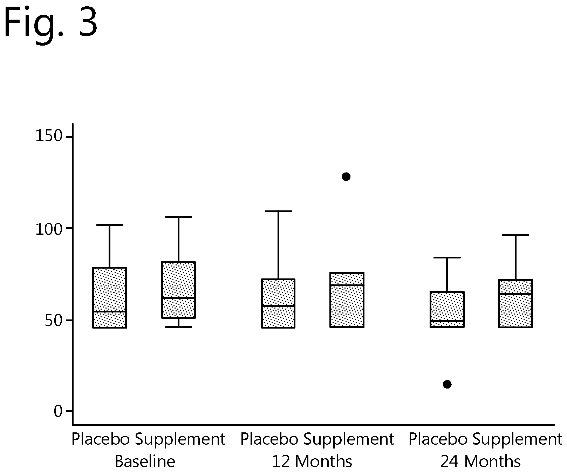

The performance of the same children on motor development scores is shown in figure 3, mean motor composite Bayley III scores are shown in table 6. A clinically relevant difference (>10) in motor development score was observed at 24 months in children that received the nutritional supplement as compared to the placebo group. A score above 85 is accepted as normal motor development.

**Table 6. Bayley III Motor composite score**

| *Visit* | *Numbers included*^{∗} | | *Mean (SD)*^{∗} | | *Difference in means (95% CI)*^{∗} |
|---|---|---|---|---|---|
| | *Placebo* | *Supplement* | *Placebo* | *Supplement* | |
| Baseline | 18 | 14 | 62.5 (20.0) | 62.3 (12.9) | |
| 12 month | 18 | 14 | 61.6 (17.0) | 67.4 (21.9) | 7.0 (-1.9, 15.9) |
| 24 month | 16 | 13 | 52.3 (18.9) | 63.1 (15.9) | 10.2 (0.9, 19.6) |

| | | | | | |
|---|---|---|---|---|---|
| * All subjects included in analysis have a measurement for Bayley motor score at baseline and at least one other time point; # adjusted for baseline measurement, treatment, visit, interaction between treatment and visit, and the minimisation factors gender, cerebral palsy type, visual impairment and corrected age at time of recruitment. | | | | | |

### Example 2 - Products for use in the treatment of global developmental delay:

An exemplary product of the invention is a liquid formula comprising the active components according to table 7. It is a bottle of infant formula for use in children of 1 year and older, that provides additionally 450 mg DHA, 126 mg choline and 21.6 mg UMP.

**Table 7.**

| *Nutrients* | **(200ml bottle) nutritional value** |
|---|---|
| **MACRO NUTRIENTS** | |
| Energy | 75 kcal/100 ml |
| Protein | 2.5-2.6 g/100 kcal |
| Whey | 0.5 (20%) g/100 kcal |
| Fat | 4.7 g/100 kcal |
| Linoleic acid | 575-1060 mg/100 kcal |
| Alpha-linolenic acid | 109-205 mg/100 kcal |
| AA | 34 mg /100 kcal |
| EPA | 60 mg/100 kcal |
| DHA | 300 mg/100 kcal |
| Carbohydrates | 11.9-12.0 g /100 kcal |
| **Sugars** | ≤5.0 g / 100 kcal |
| **MF** | 1.0-2.0 g / 100 kcal |

| **MINERALS** | |
|---|---|
| Na | 44 mg / 100 kcal |
| K | 92 mg / 100 kcal |
| Cl | 66 mg / 100 kcal |
| **Ca** | **55-88 mg / 100 kcal** |
| **P** | **50-67 mg / 100 kcal** |
| **Mg** | **11.1-14 mg / 100 kcal** |

| **TRACE ELEMENTS** | |
|---|---|
| Fe | 1.0 mg / 100 kcal |
| Zn | 7.1 mg / 100 kcal |
| Cu | 75 µg / 100 kcal |
| Mn | 65 µg / 100 kcal |
| Mo | 4.0 µg / 100 kcal |
| Se | 3.0 µg / 100 kcal |
| Cr | 2.5 µg / 100 kcal |
| I | 130 µg / 100 kcal |

| **VITAMINS** | |
|---|---|
| Vitamin A | 41 µg RE/ 100 kcal |
| Vitamin D3 | 1.3 µg / 100 kcal |
| Vitamin E | 1.4-1.6 mg α-TE / 100 kcal |
| Vitamin K1 | 4.0 µg / 100 kcal |
| Thiamin (B1) | 0.15 mg / 100 kcal |
| Riboflavin (B2) | 0.16 mg / 100 kcal |
| Niacin (B3) | 1.1 mg NE/ 100 kcal |
| Pantothenic acid (B5) | 0.33 mg / 100 kcal |
| Vitamin B6 | 0.12 mg / 100 kcal |
| Folic acid | 18 µg / 100 kcal |
| Vitamin B12 | 1.1 µg / 100 kcal |
| Biotin | 3.9 µg / 100 kcal |
| Vitamin C | 10 mg / 100 kcal |
| Choline | 104 mg / 100 kcal |
| Taurine | 7.5 mg / 100 kcal |
| L-Carnitine | 1.9 mg / 100 kcal |
| Inositol | 0 mg / 100 kcal |
| Carotenoids | 0.1 mg / 100 kcal |
| UMP | 14.4 mg / 100 kcal |
| CMP | 14.4 mg / 100 kcal |
| Total nucleotides | 28.8 mg / 100 kcal |

Another nutritional composition according to the invention is a formula to be used as supplement (once daily) for infants from birth up to 12 months of age, where children should get 20 ml/kg bodyweight per day. 200ml of formula for a child of 10 kg provide additionally 378 mg DHA, 105 mg choline and18 mg UMP. An infant should receive 20ml/kg/day. Table 8 shows an exemplary serving for a child of 10 kg.

**Table 8**

| *Nutrients* | **nutritional value for a once daily formula for a 10 kg child (200 ml serving)** |
|---|---|
| **MACRO NUTRIENTS** | |
| Energy | 150 kcal (75 kcal / 100ml) |
| Protein | 2.68 g / 100 kcal (10.8 en%) |
| Fat | 5.34 g / 100 kcal |
| Linoleic acid | 691 mg / 100 kcal |
| Alpha-linolenic acid | 97.1 mg / 100 kcal |
| AA | 52 mg / 100 kcal |
| EPA | 55 mg / 100 kcal |
| DHA | 270 mg / 100 kcal |
| Carbohydrates | 9.93 g / 100 kcal |
| Lactose | 7.78 g / 100 kcal |
| GOS/long chain FOS | 0.76 g / 100 kcal |

| **MINERALS** | |
|---|---|
| Na | 37.6 mg / 100 kcal |
| K | 104 mg / 100 kcal |
| Cl | 70.1 mg / 100 kcal |
| Ca | 117 mg / 100 kcal |
| P | 63 mg / 100 kcal |
| Mg | 9.37 mg / 100 kcal |

| **TRACE ELEMENTS** | |
|---|---|
| Fe | 1.61 mg / 100 kcal |
| Zn | 6.3 mg / 100 kcal |
| Cu | 80.2 µg / 100 kcal |
| Mn | 13.4 µg / 100 kcal |
| Se | 2.28 µg / 100 kcal |
| I | 127 µg / 100 kcal |

| **VITAMINS** | |
|---|---|
| Vitamin A | 134 µg RE / 100 kcal |
| Vitamin D3 | 2.28 µg / 100 kcal |
| Vitamin E | 2.95 mg α-TE / 100 kcal |
| Vitamin K1 | 7.91 µg / 100 kcal |
| Thiamin (B1) | 0.12 mg / 100 kcal |
| Riboflavin (B2) | 0.2 mg / 100 kcal |
| Niacin (B3) | 1.64 mg NE/ 100 kcal |
| Pantothenic acid (B5) | 0.8 mg / 100 kcal |
| Vitamin B6 | 0.11 mg / 100 kcal |
| Folic acid | 26.8 µg / 100 kcal |
| Vitamin B12 | 1.1 µg / 100 kcal |
| Biotin | 4.02 µg / 100 kcal |
| Vitamin C | 16.1 mg / 100 kcal |
| Choline | 87 mg / 100 kcal |
| Taurine | 6.57 mg / 100 kcal |
| L-Carnitine | 1.21 mg / 100 kcal |
| Inositol | 29.5 mg / 100 kcal |
| UMP | 12.9 mg / 100 kcal |
| CMP | 14.2 mg / 100 kcal |
| Total nucleotides | 28.4 mg / 100 kcal |

Another example of a nutritional composition according to the invention is a formula for infants from birth up to 12 months of age, as shown in table 9, to be used as complete nutrition.

**Table 9**

| *Nutrients* | **nutritional value for a formula for use as complete nutrition** |
|---|---|
| **MACRO NUTRIENTS** | |
| Energy | 150 kcal (75 kcal / 100ml) |
| Protein | 2.68 g / 100 kcal (10.8 en%) |
| Fat | 5.34 g / 100 kcal |
| Linoleic acid | 691 mg / 100 kcal |
| Alpha-linolenic acid | 97.1 mg / 100 kcal |
| AA | 27.8 mg / 100 kcal |
| EPA | 10.8 mg / 100 kcal |
| DHA | 51.4 mg / 100 kcal |
| Carbohydrates | 9.93 g / 100 kcal |
| Lactose | 7.78 g / 100 kcal |
| GOS/long chainFOS | 0.76 g / 100 kcal |

| **MINERALS** | |
|---|---|
| Na | 37.6 mg / 100 kcal |
| K | 104 mg / 100 kcal |
| Cl | 70.1 mg / 100 kcal |
| Ca | 117 mg / 100 kcal |
| P | 63 mg / 100 kcal |
| Mg | 9.37 mg / 100 kcal |

| **TRACE ELEMENTS** | |
|---|---|
| Fe | 1.61 mg / 100 kcal |
| Zn | 1.88 mg / 100 kcal |
| Cu | 80.2 µg / 100 kcal |
| Mn | 13.4 µg / 100 kcal |
| Se | 2.28 µg / 100 kcal |
| I | 40.1 µg / 100 kcal |

| **VITAMINS** | |
|---|---|
| Vitamin A | 134 µg RE / 100 kcal |
| Vitamin D3 | 2.28 µg / 100 kcal |
| Vitamin E | 2.95 mg α-TE / 100 kcal |
| Vitamin K1 | 7.91 µg / 100 kcal |
| Thiamin (B1) | 0.12 mg / 100 kcal |
| Riboflavin (B2) | 0.2 mg / 100 kcal |
| Niacin (B3) | 1.64 mg NE/ 100 kcal |
| Pantothenic acid (B5) | 0.8 mg / 100 kcal |
| Vitamin B6 | 0.11 mg / 100 kcal |
| Folic acid | 26.8 µg / 100 kcal |
| Vitamin B12 | 0.41 µg / 100 kcal |
| Biotin | 4.02 µg / 100 kcal |
| Vitamin C | 16.1 mg / 100 kcal |
| Choline | 26.7 mg / 100 kcal |
| Taurine | 6.57 mg / 100 kcal |
| L-Carnitine | 1.21 mg / 100 kcal |
| Inositol | 29.5 mg / 100 kcal |
| UMP | 2.47 mg / 100 kcal |
| CMP | 3.76 mg / 100 kcal |
| Total nucleotides | 7.51 mg / 100 kcal |

### Example 3. Compositions / products

Referring to Table 10. The ranges are per 100 kcal and/or per 100 ml; more preferably per 100 kcal.

**Table 10. Compositions of the invention, expressed per 100 kcal or per 100 ml product.**

| Nutrients | | | per 100 kcal and/or 100 ml. |
|---|---|---|---|
| UMP | | | 10 - 200 mg |
| Choline | | | 60 - 350 mg |
| DHA + EPA | | | 250 - 9000 mg |
| DHA | | | 250 - 900 mg |
| EPA | | | 50 - 150 mg |
| vitamin B12 | | | 1 - 5 mcg |
| iodine | | | 60 - 150 mcg |
| zinc | | | 5 - 25 mg |

## Claims

1. A composition comprising therapeutically effective amounts of (i) one or more of uridine and cytidine, or salts, phosphates, acyl derivatives or esters thereof, (ii) at least one of docosahexaenoic acid (22:6; DHA), eicosapentaenoic acid (20:5; EPA) and docosapentaenoic acid (22:5; DPA), or esters thereof, (iii) choline, or salts or esters thereof, for use in therapeutically treating or preventing global developmental delay in a child, wherein said composition comprises:
(i) 10 - 200 mg uridine monophosphate (UMP) per 100 kcal or per 100 ml; and
(ii) 250 - 9000 mg DHA + EPA per 100 kcal or per 100 ml; and
(iii) 60 - 350 mg choline per 100 kcal or per 100 ml; and
(iv) 1 - 5 µg vitamin B12 or equivalents per 100 kcal or per 100 ml, and
(v) per 100 kcal or per 100 ml, 60 - 150 µg of iodine and 5-25 mg of zinc.

2. The composition for use according to any one of the preceding claims, wherein the child is at risk of or suspected of having cerebral palsy and/or has an age between 1 month and 12 years, preferably between 1 and 42 months.

3. The composition for use according to any one of the preceding claims, wherein the child at risk of cerebral palsy was born preterm and small for gestational age; or born preterm with brain injury; or born term but small for gestational age; or born term with brain injury, or born preterm and small for gestational age with brain injury or born term but small for gestational age with brain injury.

4. The composition for use according to any one of the preceding claims, wherein DHA is administered in a daily amount of 32.5 - 65 mg/kg body weight, preferably 35 - 62.0 mg/kg body weight, more preferably 36-60 mg/kg body weight, even more preferably 36-50 mg/kg bodyweight, most preferably 36-41 mg/kg body weight.

5. The composition for use according to any one of the preceding claims, wherein said child prior to treatment has a Bayley III cognitive composite score of less than 85.

6. The composition for use according to any one of the preceding claims, wherein the total daily dosage of DHA+EPA+DPA is in the range of 35 - 75 mg/kg body weight, preferably 40 - 72.5 mg/kg body weight, preferably 43.5 - 72.5 mg/kg body weight, preferably 43.5 - 60 mg/kg body weight, more preferably 43 - 49.5 mg/kg bodyweight.

7. The composition for use according to any one of the preceding claims, wherein uridine, as the cumulative amount of uridine, deoxyuridine, uridine phosphates, nucleobase uracil and acylated uridine derivatives, is administered in a daily amount of 1.0 - 4.0 mg per kg body weight, preferably 1.0 - 3.5 mg per kg body weight, more preferably 1.5 - 3.0 mg per kg body weight, more preferably 1.8 - 3.0 mg/kg body weight, even more preferably 1.8 - 2.0 mg/kg body weight.

8. The composition for use according to any one of the preceding claims, wherein the total daily dosage of choline, preferably as choline salt, is 7.5 to 35 mg, more preferably 8 to 31 mg, more preferably 9-31 mg per kg body weight, most preferably 9 to 14 mg choline per kg body weight.

## Patentansprüche

1. Zusammensetzung, umfassend therapeutisch wirksame Mengen von (i) einem oder mehreren von Uridin und Cytidin oder Salzen, Phosphaten, Acylderivaten oder Estern dieser, (ii) mindestens einem von Docosahexaensäure (22:6; DHA), Eicosapentaensäure (20:5; EPA) und Docosapentaensäure (22:5; DPA) oder Estern dieser, (iii) Cholin oder Salzen oder Estern dieser, zur Anwendung bei der therapeutischen Behandlung oder Vorbeugung einer globalen Entwicklungsverzögerung bei einem Kind, wobei die Zusammensetzung Folgendes umfasst:
(i) 10 - 200 mg Uridinmonophosphat (UMP) pro 100 kcal oder pro 100 mL; und
(ii) 250 - 9000 mg DHA+EPA pro 100 kcal oder pro 100 mL; und
(iii) 60-350 mg Cholin pro 100 kcal oder pro 100 mL; und
(iv) 1 - 5 µg Vitamin B12 oder Äquivalente pro 100 kcal oder pro 100 mL, und
(v) pro 100 kcal oder pro 100 mL, 60-150 µg Jod und 5-25 mg Zink.

2. Zusammensetzung zur Anwendung nach einem der voranstehenden Ansprüche, wobei das Kind gefährdet ist oder im Verdacht steht, zerebrale Lähmung zu haben, und/oder ein Alter zwischen 1 Monat und 12 Jahren, vorzugsweise zwischen 1 und 42 Monaten, hat.

3. Zusammensetzung zur Awendung nach einem der voranstehenden Ansprüche, wobei das Kind mit dem Risiko einer zerebralen Lähmung als Frühgeburt und klein für das Gestationsalter geboren wurde; oder als Frühgeburt mit einer Hirnschädigung geboren wurde; oder termingerecht, aber klein für das Gestationsalter geboren wurde; oder termingerecht mit einer Hirnschädigung geboren wurde, oder als Frühgeburt und klein für das Gestationsalter mit einer Hirnschädigung geboren wurde oder termingerecht, aber klein für das Gestationsalter mit einer Hirnschädigung geboren wurde.

4. Zusammensetzung zur Anwendung nach einem der voranstehenden Ansprüche, wobei DHA in einer täglichen Menge von 32,5 - 65 mg/kg Körpergewicht, vorzugsweise 35 - 62,0 mg/kg Körpergewicht, noch bevorzugter 36-60 mg/kg Körpergewicht, noch bevorzugter 36-50 mg/kg Körpergewicht, am meisten bevorzugt 36-41 mg/kg Körpergewicht verabreicht wird.

5. Zusammensetzung zur Anwendung nach einem der voranstehenden Ansprüche, wobei das Kind vor der Behandlung eine Bayley-III kognitive Gesamtbewertung von weniger als 85 hat.

6. Zusammensetzung zur Anwendung nach einem der voranstehenden Ansprüche, wobei die tägliche Gesamtdosis von DHA+EPA+DPA im Bereich von 35 - 75 mg/kg Körpergewicht, vorzugsweise 40 - 72,5 mg/kg Körpergewicht, vorzugsweise 43,5 - 72,5 mg/kg Körpergewicht, vorzugsweise 43,5 - 60 mg/kg Körpergewicht, noch bevorzugter 43 - 49,5 mg/kg Körpergewicht liegt.

7. Zusammensetzung zur Anwendung nach einem der voranstehenden Ansprüche, wobei Uridin, als die kumulative Menge von Uridin, Desoxyuridin, Uridinphosphaten, Nukleobase Uracil und acylierten Uridinderivaten, in einer täglichen Menge von 1,0 - 4,0 mg pro kg Körpergewicht, vorzugsweise 1,0 - 3,5 mg pro kg Körpergewicht, noch bevorzugter 1,5 - 3,0 mg pro kg Körpergewicht, noch bevorzugter 1,8 - 3,0 mg/kg Körpergewicht, noch bevorzugter 1,8 - 2,0 mg/kg Körpergewicht verabreicht wird.

8. Zusammensetzung zur Anwendung nach einem der voranstehenden Ansprüche, wobei die tägliche Gesamtdosis an Cholin, vorzugsweise als Cholinsalz, 7,5 bis 35 mg, vorzugsweise 8 bis 31 mg, besonders bevorzugt 9-31 mg pro kg Körpergewicht, am meisten bevorzugt 9 bis 14 mg Cholin pro kg Körpergewicht beträgt.

## Revendications

1. Composition comprenant des quantités thérapeutiquement efficaces de (i) un ou plusieurs parmi l'uridine et la cytidine, ou des sels, des phosphates, des dérivés d'acyle ou des esters de celles-ci, (ii) au moins un parmi l'acide docosahexaénoïque (22:6 ; DHA), l'acide eicosapentaénoïque (20:5 ; EPA) et l'acide docosapentaénoïque (22:5 ; DPA), ou des esters de ceux-ci, (iii) la choline, ou des sels ou esters de celle-ci, à utiliser dans le traitement thérapeutique ou la prévention d'un retard de développement global chez un enfant, dans laquelle ladite composition comprend :
i) de 10 à 200 mg d'uridine monophosphate (UMP) pour 100 kcal ou pour 100 ml ; et
ii) de 250 à 9 000 mg de DHA + EPA pour 100 kcal ou pour 100 ml ; et
(iii) de 60 à 350 mg de choline pour 100 kcal ou pour 100 ml ; et
(iv) de 1 à 5 µg de vitamine B12 ou d'équivalents pour 100 kcal ou 100 ml, et
(v) pour 100 kcal ou pour 100 ml, de 60 à 150 µg d'iode et de 5 à 25 mg de zinc.

2. Composition à utiliser selon l'une quelconque des revendications précédentes, dans laquelle l'enfant est à risque ou soupçonné d'avoir une paralysie cérébrale et/ou a un âge compris entre 1 mois et 12 ans, de préférence entre 1 et 42 mois.

3. Composition à utiliser selon l'une quelconque des revendications précédentes, dans laquelle l'enfant à risque de paralysie cérébrale est né prématuré et petit pour l'âge gestationnel ; ou est né prématuré avec une lésion cérébrale ; ou est né à terme mais petit pour l'âge gestationnel ; ou est né à terme avec une lésion cérébrale, ou est né prématuré et petit pour l'âge gestationnel avec une lésion cérébrale ou est né à terme mais petit pour l'âge gestationnel avec une lésion cérébrale.

4. Composition à utiliser selon l'une quelconque des revendications précédentes, dans laquelle le DHA est administré en une quantité quotidienne de 32,5 à 65 mg/kg de poids corporel, de préférence de 35 à 62,0 mg/kg de poids corporel, de manière plus préférée de 36 à 60 mg/kg de poids corporel, de manière encore plus préférée de 36 à 50 mg/kg de poids corporel, de manière la plus préférée de 36 à 41 mg/kg de poids corporel.

5. Composition à utiliser selon l'une quelconque des revendications précédentes, dans laquelle ledit enfant, avant le traitement, présente un score composite cognitif de Bayley III inférieur à 85.

6. Composition à utiliser selon l'une quelconque des revendications précédentes, dans laquelle la dose quotidienne totale de DHA + EPA + DPA est dans la plage de 35 à 75 mg/kg de poids corporel, de préférence de 40 à 72,5 mg/kg de poids corporel, de préférence de 43,5 à 72,5 mg/kg de poids corporel, de préférence de 43,5 à 60 mg/kg de poids corporel, de manière plus préférée de 43 à 49,5 mg/kg de poids corporel.

7. Composition à utiliser selon l'une quelconque des revendications précédentes, dans laquelle l'uridine, sous forme de quantité cumulée d'uridine, de désoxyuridine, de phosphates d'uridine, d'uracile nucléobase et de dérivés d'uridine acylés, est administrée en une quantité quotidienne de 1,0 à 4,0 mg par kg de poids corporel, de préférence de 1,0 à 3,5 mg par kg de poids corporel, de manière plus préférée de 1,5 à 3,0 mg par kg de poids corporel, de manière plus préférée de 1,8 à 3,0 mg/kg de poids corporel, de manière encore plus préférée de 1,8 à 2,0 mg/kg de poids corporel.

8. Composition à utiliser selon l'une quelconque des revendications précédentes, dans laquelle la dose quotidienne totale de choline, de préférence sous forme de sel de choline, est de 7,5 à 35 mg, de manière plus préférée de 8 à 31 mg, de manière plus préférée de 9 à 31 mg par kg de poids corporel, de la manière la plus préférée de 9 à 14 mg de choline par kg de poids corporel.
